# EUROPEAN PATENT APPLICATION

(11) **EP 2 769 690 A1**
(43) Date of publication of application: **27.08.2014**
(21) Application number: 14155878.3
(22) Date of filing: 20.02.2014
(51) Int. Cl.: A61B 17/34, A61B 19/00

(54) **Optical obturator visualization system**

(30) Priority: 21.02.2013 US 201361767426 P; 15.01.2014 US 201414155607
(71) Applicant: Covidien LP, Mansfield, MA 02048 (US)
(72) Inventor: Smith, Robert C., Middlefield, CT Connecticut 06455 (US)
(74) Representative: Soames, Candida Jane

(57) **Abstract**

A visualization system includes an obturator assembly and an endoscope. The obturator assembly includes two body halves defining a longitudinal split line. Each of the two body halves includes a housing portion and an elongate member portion extending distally from the housing portion. The two body halves define a longitudinal bore extending between proximal and distal ends thereof. The endoscope is configured for insertion into the longitudinal bore of the obturator assembly, wherein at least a portion of the obturator assembly is transparent to permit visualization of tissue with the endoscope.

## Description

### CROSS-REFERENCE TO RELATED APPLICATIONS

This application claims the benefit of and priority to U.S. Provisional Patent Application No. 61/767,426, filed February 21, 2013, the entire disclosure of which is incorporated by reference herein.

### TECHNICAL FIELD

The present disclosure relates to a visualization system for tunneling through body tissue and, more particularly, to an optical obturator assembly including two transparent body halves, which facilitate penetration of body tissue under direct observation.

### BACKGROUND OF RELATED ART

Endoscopic and laparoscopic minimally invasive procedures have been used for introducing medical devices into a patient and for viewing portions of the patient's anatomy. Typically, to view a desired anatomical site, a surgeon inserts an endoscope inside the patient to render images of the anatomical site. In endoscopic surgical procedures, surgery is performed in any hollow organ or tissue of the body through a small incision or through narrow endoscopic tubes (cannulas) inserted through a small entrance wound in the skin. In laparoscopic procedures, surgical operations in the abdomen are performed through small incisions (usually about 0.5 cm to about 1.5 cm). Laparoscopic and endoscopic procedures often require the surgeon to act on organs, tissues and vessels far removed from the incision, thereby requiring that any instruments used in such procedures be of sufficient size and length to permit remote operation.

Typically, a trocar is used to puncture the body cavity and includes a cannula which remains in place for use during the laparoscopic procedure. Generally, a trocar includes a stylet or obturator for penetrating the body cavity.

Therefore, a need exists for a cost effective, rigid obturator that is also easy to clean and sterilize.

### SUMMARY

There is provided a visualization system in accordance with an embodiment of the present disclosure. The visualization system includes an optical obturator assembly and an endoscope. The optical obturator assembly includes two body halves defining a longitudinal split line. Each of the two body halves includes a housing portion and an elongate member portion extending distally from the housing portion. The two body halves define a longitudinal bore extending between proximal and distal ends thereof. The endoscope is configured for insertion into the longitudinal bore of the optical obturator assembly, wherein at least a portion of the optical obturator assembly is transparent to permit visualization of tissue with the endoscope.

In an embodiment, the two body halves may define a blunt end portion configured for separation of tissue planes and blunt dissection of cavity linings. In addition, the optical obturator assembly may include indicia to guide longitudinal alignment of the two body halves. In particular, the two body halves may be substantially identical.

In yet another embodiment, the optical obturator assembly may further include a shrink wrap configured to secure the two body halves together as a single construct. The shrink wrap may be wrapped around the elongate member portions of the two body halves.

In still another embodiment, one of the two body halves may include a hook portion on a longitudinal edge thereof, and the other of the two body halves may include a slot portion configured to receive the hook portion therein to secure the two body halves together.

In still yet another embodiment, the elongate member portion may have a uniform thickness. The elongate member portions of the two body halves may be transparent. In particular, the two body halves may be entirely transparent.

In other embodiments, the visualization system may further include a cannula assembly including a cannula housing and a cannula sleeve extending distally from the cannula housing. The cannula assembly may define a longitudinal channel extending between a proximal end of the cannula assembly to a distal end of the cannula assembly. The longitudinal channel may be configured and dimensioned to receive at least a portion of the optical obturator assembly therethrough.

In various embodiments, the present invention may relate to an obturator assembly comprising: a first obturator body half having a first housing portion and a first elongate member portion extending distally from the housing portion; a second obturator body half having a second housing portion and a second elongate member portion extending distally from the housing portion; the first and second obturator body halves configured to be joined so as to form an obturator, wherein, when the first and second obturator body halves are joined, the first and second housing portions define a housing and the first and second elongate member portions define an elongate tubular member having a longitudinal bore extending between proximal and distal ends thereof, the housing and longitudinal bore configured to receive an endoscope. The elongate tubular member may be at least partially transparent.

In various embodiments, the present invention may relate to a method of manufacturing an obturator assembly comprising the steps of: molding a first obturator body half having a first housing portion and a first elongate member portion extending distally from the housing portion; molding a second obturator body half having a second housing portion and a second elongate member portion extending distally from the housing portion; joining the first and second obturator body halves so as to form an obturator, wherein, when the first and second obturator body halves are joined, the first and second housing portions define a housing and the first and second elongate member portions define an elongate tubular member having a longitudinal bore extending between proximal and distal ends thereof. The method may also include the step of inserting an endoscope through the housing and the longitudinal bore. The molding steps may include molding the first and second elongate member portions from a material that is at least partially transparent.

### BRIEF DESCRIPTION OF THE DRAWINGS

The above and other aspects, features, and advantages of the present disclosure will become more apparent in light of the following detailed description when taken in conjunction with the accompanying drawings in which:
FIG. 1 is a perspective view of a surgical visualization system with parts separated in accordance with an embodiment of the present disclosure;
FIG. 2 is a perspective view of the surgical visualization system of FIG. 1 assembled for use;
FIG. 3 is a front view of one of two body halves of an optical obturator assembly of the surgical visualization system of FIG. 1;
FIG. 4 is a top view of the optical obturator assembly of FIG. 1;
FIG. 5 is partial side view of one of the two body halves of an optical obturator assembly for use with the surgical visualization system of FIG. 1 in accordance with another embodiment of the present disclosure;
FIG. 6 is a partial front view of the one of the two body halves of the optical obturator assembly in FIG. 5; and
FIG. 7 is a partial front view of the other of the two body halves corresponding to the one of the two body halves of FIG. 5.

### DETAILED DESCRIPTION

Embodiments of the present disclosure will now be described in detail with reference to the drawings, in which like reference numerals designate identical or corresponding elements in each of the several views. As used herein, the term "distal," as is conventional, will refer to that portion of the instrument, apparatus, device or component thereof which is farther from the user while, the term "proximal," will refer to that portion of the instrument, apparatus, device or component thereof which is closer to the user. In the following description, well-known functions or constructions are not described in detail to avoid obscuring the present disclosure in unnecessary detail.

Various embodiments of the surgical visualization system disclosed herein may be employed in endoscopic, laparoscopic, open surgical procedures, and interventional and intralumenal procedures such as GI sheathing (metabolic/bariatric) and/or banding. In addition, the system of the present disclosure may be utilized for post-operative monitoring, diagnostics and combinations thereof.

Various embodiments of the visualization system of the present disclosure may include devices inserted in a patient to provide visualization of the target site. These devices may be introduced into the patient using minimally invasive procedures through natural orifices such as, e.g., navel, vagina and/or anus, or via a device inserted through a trocar, for example, and may be adapted to provide images of the surgical site or anatomic location such as the lungs, liver, stomach, gall bladder, urinary tract, reproductive tract, and intestinal tissue, for example. Once positioned at the target site, the surgical visualization devices provide images that enable the surgeon to more accurately diagnose and provide more effective treatment of the diseased tissue. In embodiments, the visualization apparatus may be inserted into the tissue treatment region percutaneously. In other embodiments, the surgical visualization device may be introduced into the tissue treatment region endoscopically (e.g., laparoscopically and/or thoracoscopically), through small keyhole incisions via a trocar, or through a natural orifice.

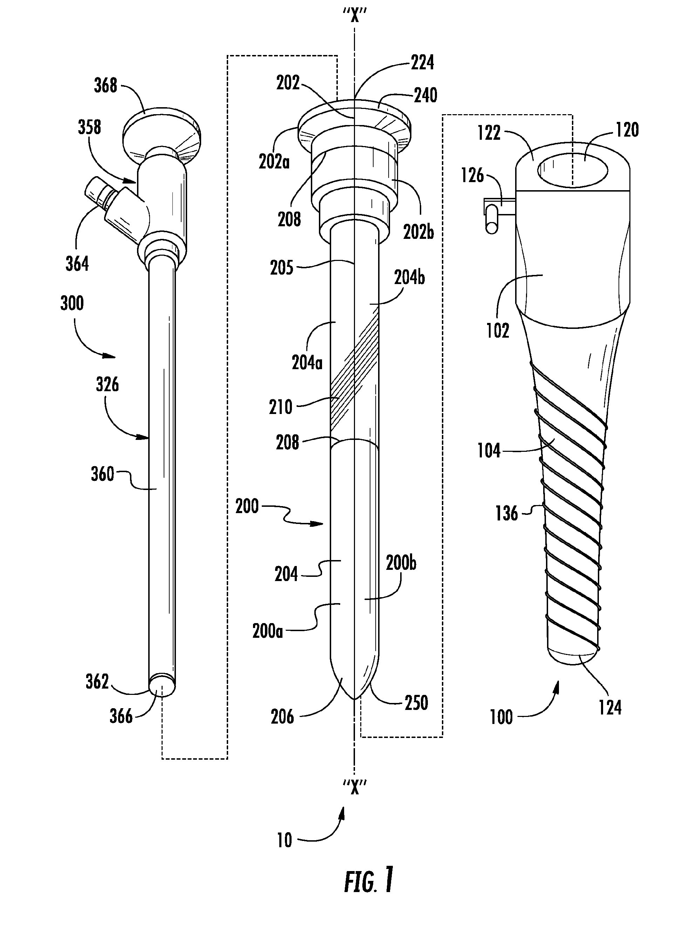

With reference now to FIG. 1, there is illustrated a surgical visualization system 10 in accordance with an embodiment of the present disclosure. System 10 includes a cannula assembly 100, an optical obturator assembly 200, and an endoscope 300. Cannula assembly 100 is configured and dimensioned to receive optical obturator assembly 200 therethrough, and optical obturator assembly 200 is configured to receive endoscope 300 therein.

Cannula assembly 100 serves as an access port for optical obturator assembly 200 and endoscope 300. Moreover, cannula assembly 100 provides a conduit for a supply of insufflation fluid to insufflate the body cavity to create working space. Cannula assembly 100 includes a cannula housing 102 and an elongate cannula sleeve 104 extending distally from cannula housing 102. Cannula housing 102 is configured to mechanically engage a proximal portion of elongate cannula sleeve 104. Cannula housing 102 and cannula sleeve 104 define a through bore 120 that extends from a proximal end 122 of cannula housing 102 to a distal end 124 of cannula sleeve 104. Additionally, a valve 126 is provided on cannula housing 102 to be coupled with a fluid source (not shown) to provide insufflation fluid into the body cavity of a patient.

Elongate cannula sleeve 104 includes a plurality of ribs 136 extending radially inward from an inner surface of cannula sleeve 104 and radially outward from an outer surface of cannula sleeve 104. The plurality of ribs 136 are configured to enhance securement of cannula assembly 100 within tissue tract, as well as securement of optical obturator assembly 200 within through bore 120. Cannula housing 102 may include seals such as, e.g., an insert seal and a zero-closure seal (not shown), to maintain the insufflation fluid in the body cavity. These components may be assembled together to define a single unit which may be subsequently attached to elongate cannula sleeve 104 of cannula assembly 100.

With continued reference to FIG. 1, optical obturator assembly 200 is configured to penetrate tissue and permit visualization of tissue to the surgeon performing the procedure. Optical obturator assembly 200 includes an obturator housing 202 and an elongate member 204 extending distally from obturator housing 202. Elongate member 204 may be substantially hollow in structure to provide a passageway for endoscope 300, as will be discussed hereinbelow.

Elongate member 204 defines a bladeless tip or a blunt end portion 206 for separating tissue planes and blunt dissection of cavity linings during a surgical procedure. Blunt end portion 206 permits initial insertion within an opening, e.g., a pre-cut scalpel incision, in tissue and facilitates advancement of elongate member 204 between the tissue layers to gently dissect tissue, without any cutting or incising of the tissue. After initial insertion and continued distal insertion, tubular portion 210 of elongate member 204 continues to gently enlarge the opening in tissue.

Optical obturator assembly 200 is made of a transparent material to provide visualization of the tissue tract and the body cavity through endoscope 300. In particular, obturator assembly 200 is transparent throughout its entire length. Obturator assembly 200 may be constructed from a number of transparent materials such as, e.g., glass, acrylic glass, polystyrene, or polycarbonate.

In particular, interior and exterior surfaces of blunt end portion 206 are refracting surfaces that are adapted to direct light along a predetermined path, e.g., along a direction substantially parallel to a longitudinal axis "X-X." This arrangement provides for generally direct or forward viewing and illumination of the surgical site.

Elongate member 204 of optical obturator assembly 200 includes a wall having a substantially uniform thickness. The uniform thickness reduces distortion of an image observed therethrough. Furthermore, the wall of the elongate member 204 is relatively thin. In embodiments, the wall thicknesses of elongate member 204 may be from about 0.02 inches (about 0.5 mm) to about 0.025 inches (about 0.65 mm) for about a 5 mm to about 12 mm access apparatus. The thin-wall configuration enables light to travel through the material with reduced loss in intensity, thereby enhancing the visibility of tissue through elongate member 204 as optical obturator assembly 200 is advanced and placed into the targeted body cavity. The thin-wall configuration also reduces distortion of the image viewed through elongate member 204 and maintains the color accuracy of the viewed tissue.

Optical obturator assembly 200 includes two body halves 200a, 200b. Two body halves 200a, 200b are substantially identical to each other. Two body halves 200a, 200b are shrink wrapped together to form a unitary construct. For example, the shrink wrap may be applied to only a portion of obturator housing 202 such as, e.g., a tubular portion 210, of elongate member 204 to further simplify the process. Moreover, the shrink wrap may be positioned such that visibility of tissue through two body halves 200a, 200b is not affected.

Two body halves 200a, 200b define a longitudinal split line 205 extending from a proximal end portion 240 of obturator housing 202 to a distal end portion 250 of elongate member 204. Construction of substantially identical two body halves 200a, 200b, through, e.g., molding, rather than forming a single body construct significantly simplifies the manufacture of obturator assembly 200, which, in turn, reduces costs of production. For example, molding an optical obturator as a single body provides challenges in that a core pin is required in order to form the inner surface of the obturator, and maintaining that core pin in its precise location during the entire molding process (in order to ensure that the wall thickness of the obturator remains uniform) typically requires that core support pins be used to support the core pin. The molding tools having these core pins and core support pins are necessarily more complex and expensive than the more simplistic molds that can be employed in accordance with various embodiments of the present invention. In addition, these core support pins leave openings through the obturator wall that, when the product is eventually used in surgery, permit the passage of insufflation gas threrethrough, which may not be desirable.

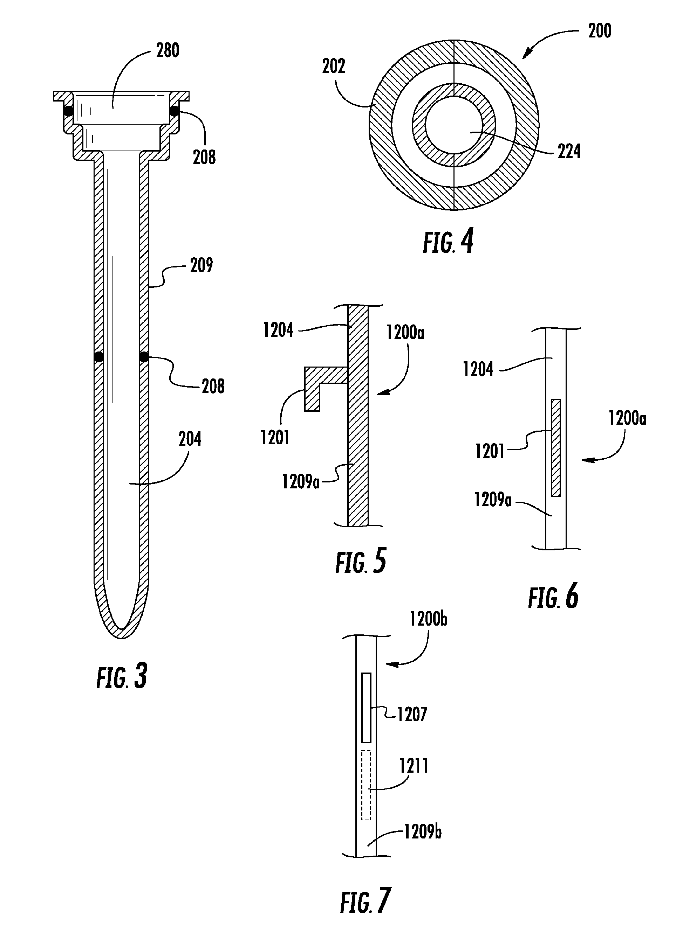

Obturator assembly 200 may be disposable after use or reusable. If reusable, obturator assembly 200 may be sterilized for subsequent use. Utilizing shrink wrap enables the clinician to easily separate two body halves 200a, 200b. The separability of two body halves 200a, 200b improves and facilitates sterilization of obturator assembly 200. An edge 209 (FIG. 3) of elongate member 204 may be chamfered (not shown) such that when edges of 209 of two body halves 200a, 200b are in contact to form a unitary construct, edges define a recess resulting in a gap between the shrink wrap and the recess. The gap enables the clinician to insert, e.g., a knife, therein to cut through the shrink wrap to separate two body halves 200a, 200b, prior to sterilization of obturator assembly 200.

In order to facilitate alignment of two body halves 200a, 200b, e.g., prior to application of the shrink wrap, obturator assembly 200 includes a lateral indicia 208 on each of two body halves 200a, 200b to visually guide the clinician in aligning two body halves 200a, 200b together.

Each of the two body halves 200a, 200b is monolithically formed such that each of the two body halves 200a, 200b is entirely transparent. However, only elongate member portions 204a, 204b may be transparent and obturator housing portions 202a, 202b may be formed of a non-transparent material such as, e.g., stainless steel, titanium and/or alloys thereof, polymeric materials, and ceramics. Some embodiments of obturator assembly 200 may further include a composite, for example, a fiber-reinforced polymer. In some embodiments, a stronger material permits reduction in a wall thickness of a component without compromising the strength thereof.

Alternatively, two body halves 200a, 200b may be glued or welded together to form the obturator assembly. With reference now to FIGS. 5-7, it is also envisioned that two body halves 1200a, 1200b may include an engaging mechanism. One of the two body halves 1200a may include a hook portion 1201 on an edge 1209a, and the other of the two body halves 1200b may include a corresponding slot 1211 shown in phantom (FIG. 7) having an opening 1207 configured and dimensioned to receive hook portion 1201 therein. Under such a configuration, hook portion 1201 may be received in opening 1207 and thereafter slidably positioned in slot 1211 such that two body halves 1200b are secured with each other. Furthermore, such a configuration also aids aligning of two body halves 1200a, 1200b together. It is further contemplated that the engaging mechanism may be used in conjunction with the shrink wrap to further secure two body halves 1200a, 1200b together.

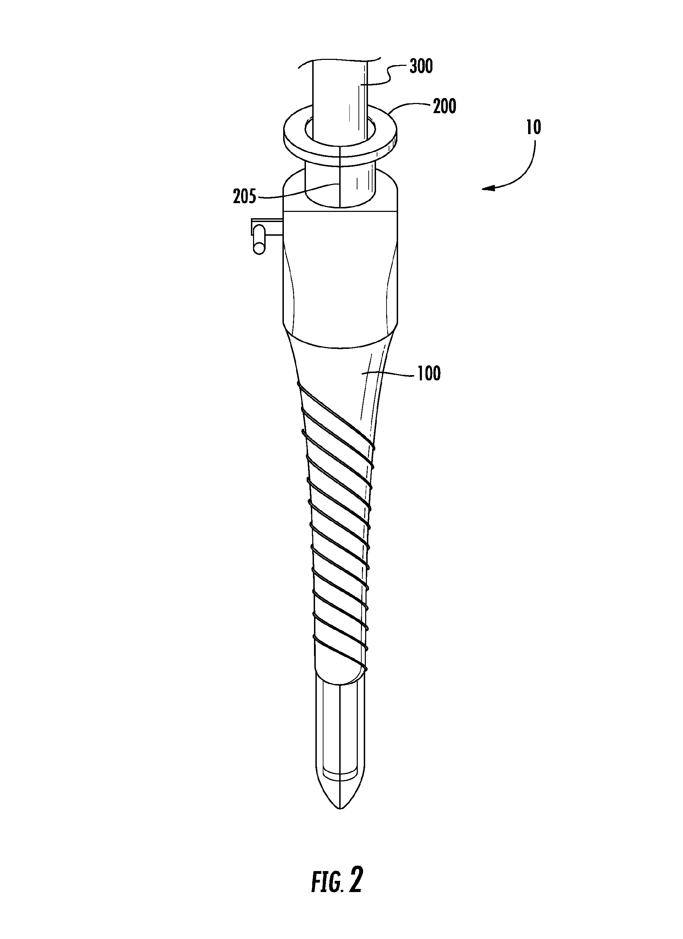

With reference back to FIGS. 1 and 2, obturator assembly 200 is secured with cannula assembly 100 through a friction fit engagement of obturator housing 202 in through bore 120 defined in cannula housing 102. However, cannula housing 102 and obturator housing 202 may include other retention mechanisms for a releasable attachment such as, e.g., bayonet coupling, threaded connection, latch, tongue and groove arrangement, and snap-fit.

Obturator housing 202 includes an opening 280 (FIG. 3) and a scope retention member (not shown) adjacent opening 280. The scope retention member is adapted to engage the outer surface of endoscope 300 in a frictional engagement therewith to assist in retaining the relative positioning of endoscope 300 within obturator assembly 200.

With particular reference to FIG. 1, endoscope 300 may be any conventional scope suitable for endoscopic applications including, e.g., a laparoscope, arthroscope, colonoscope, etc. Endoscope 300 may incorporate an optical train or lens arrangement which is capable of transmitting an image of an object from the distal or objective lens through the eyepiece or monitor for viewing by the surgeon.

Endoscope 300 includes an endoscopic portion 326 and an endoscope housing 358. Endoscopic portion 326 is configured to transfer illuminating light from endoscope housing 358 to the distal end of endoscopic portion 326 to provide illuminating light to the operative site. Endoscopic portion 326 includes an outer sheath 360 and an annular array of fiber optic elements 362 extending between light source connector 364 of endoscope housing 358 and the distal end of outer sheath 360 to illuminate the operative site. Any known light source may be connected to connector 364 to provide the illuminating light. In addition, endoscopic portion 326 includes an image transferring system 366 which may include a bundle of fiber optic elements or objective lenses which transfer an optical image to eyepiece 368 for viewing. Alternatively, a video system including a monitor may be operatively connected to housing 358 to provide a video image of the body tissue being penetrated in the working area. Preferably, fiber optic elements 362 are positioned adjacent the inner wall of outer sheath 360 so as to surround image transferring system 366. In this configuration, optical images which impinge on an image directing member (not shown) are directed into the image transferring system and relayed to eyepiece 368.

Endoscope 300 may be positioned within optical obturator assembly 200 and the assembled unit may advance through an incision and into the body cavity as a single body. During the advancement within tissue, endoscope 300 permits constant visualization of the neighboring tissue thereby providing confirmation upon entering into the body cavity while also minimizing undesired contact or engagement with any underlying organs or other body tissues. Alternatively, endoscope 300 may be positioned within optical obturator assembly 200 after optical obturator assembly 200 has been advanced into the body cavity.

The use and function of surgical visualization system 10 will now be discussed. In embodiments, in laparoscopic surgery, the abdominal cavity is insufflated with a suitable biocompatible gas to insufflate the body cavity and lift the body cavity wall away from the internal organs therein.

In operation, an initial incision is made by, e.g., a scalpel. The incision is preferably small, for example, within a range from about 2 mm to about 7 mm. Obturator assembly 200 is at least partially introduced within cannula assembly 100. Endoscope 300 is inserted into longitudinal bore 224 of elongate member 204, as shown in FIG. 2. The surgeon then positions blunt end portion 206 of obturator assembly 200 against the body tissue and continuously moves blunt end portion 206 to dissect or separate tissue along a natural tissue plane to gain access to an underlying cavity in a non-traumatic fashion. During penetration of the body tissue the surgeon either observes such penetration through eyepiece 368, or in instances where a video system is utilized the surgeon observes the penetration of the body tissue via any known video monitor.

The surgeon may insert obturator assembly 200 and bluntly penetrate the body tissue until reaching thicker tissue, such as muscle. At this point, a blade (not shown) can be deployed to cut through this thick tissue.

Once system 10 is positioned at the desired location relative to the body cavity, endoscope 300 may be used to monitor the desired surgical procedure being performed within the cavity. Alternatively, upon penetration into the body cavity, both endoscope 300 and obturator assembly 200 may be removed from cannula assembly 100, while leaving the cannula assembly 100 in the body for insertion of desired surgical instrumentation therethrough.

It will be understood that various modifications and changes in form and detail may be made to the embodiments of the present disclosure without departing from the spirit and scope of the invention. For example, obturator assembly 200 may incorporate its own illumination and optical devices or systems. For example, obturator assembly 200 may include illumination means and image transmitting means each extending in a general longitudinal direction through elongate member 204 and terminating within blunt end portion 206. Illumination means is configured to transfer illuminating light to the operative site. Optionally, illumination means may be adjustably positionable within elongate member 204 in an axial direction, and/or in a direction transverse to the longitudinal axis, in order to selectively direct light to the surgical area. Illumination means may include fiber optics or a liquid light transferring medium. Illumination means may include a bundle of fiber optic elements or lenses which transfer an optical image for viewing by the surgeon.

Therefore, the above description should not be construed as limiting the invention but merely as exemplifications of preferred embodiments thereof. Those skilled in the art will envision other modifications within the scope and spirit of the present invention as defined by the claims appended hereto. Having thus described the invention with the details and particularity required by the patent laws, what is claimed and desired protected is set forth in the appended claims.

The invention may be described by reference to the following numbered paragraphs:-
1. A visualization system, comprising:
   an obturator assembly including two body halves defining a longitudinal split line, each of the two body halves including a housing portion and an elongate member portion extending distally from the housing portion, the two body halves defining a longitudinal bore extending between proximal and distal ends thereof; and
   an endoscope configured for insertion into the longitudinal bore of the obturator assembly, wherein at least a portion of the obturator assembly is transparent to permit visualization of tissue with the endoscope.
2. The visualization system according to paragraph 1, wherein the two body halves define a blunt end portion configured for separation of tissue planes and blunt dissection of cavity linings.
3. The visualization system according to paragraph 1, wherein the obturator assembly includes indicia to guide longitudinal alignment of the two body halves.
4. The visualization system according to paragraph 1, wherein the two body halves are substantially identical.
5. The visualization system according to paragraph 1, wherein the obturator assembly further includes a shrink wrap configured to secure the two body halves together as a single construct.
6. The visualization system according to paragraph 5, wherein the shrink wrap is wrapped around the elongate member portions of the two body halves.
7. The visualization system according to paragraph 1, wherein one of the two body halves includes a hook portion on a longitudinal edge thereof, and the other of the two body halves includes a slot portion configured to receive the hook portion therein to secure the two body halves together.
8. The visualization system according to paragraph 1, wherein the elongate member portion has a uniform thickness.
9. The visualization system according to paragraph 1, wherein the elongate member portions of the two body halves are transparent.
10. The visualization system according to paragraph 1, wherein the two body halves are entirely transparent.
11. The visualization system according to paragraph 1, further comprising a cannula assembly including a cannula housing and a cannula sleeve extending distally from the cannula housing, the cannula assembly defining a longitudinal channel extending between a proximal end of the cannula assembly to a distal end of the cannula assembly.
12. The visualization system according to paragraph 11, wherein the longitudinal channel is configured and dimensioned to receive at least a portion of the obturator assembly therethrough.
13. An obturator assembly comprising:
   a first obturator body half having a first housing portion and a first elongate member portion extending distally from the housing portion;
   a second obturator body half having a second housing portion and a second elongate member portion extending distally from the housing portion;
   the first and second obturator body halves configured to be joined so as to form an obturator, wherein, when the first and second obturator body halves are joined, the first and second housing portions define a housing and the first and second elongate member portions define an elongate tubular member having a longitudinal bore extending between proximal and distal ends thereof, the housing and longitudinal bore configured to receive an endoscope.
14. The obturator assembly according to paragraph 1, wherein the elongate tubular member is at least partially transparent.
15. A method of manufacturing an obturator assembly comprising the steps of:
   molding a first obturator body half having a first housing portion and a first elongate member portion extending distally from the housing portion;
   molding a second obturator body half having a second housing portion and a second elongate member portion extending distally from the housing portion;
   joining the first and second obturator body halves so as to form an obturator, wherein, when the first and second obturator body halves are joined, the first and second housing portions define a housing and the first and second elongate member portions define an elongate tubular member having a longitudinal bore extending between proximal and distal ends thereof.
16. The method according to paragraph 15, further comprising the steps of:
   inserting an endoscope through the housing and the longitudinal bore.
17. The method according to paragraph 15, wherein the molding steps include molding the first and second elongate member portions from a material that is at least partially transparent.

## Claims

1. A visualization system, comprising:
an obturator assembly including two body halves defining a longitudinal split line, each of the two body halves including a housing portion and an elongate member portion extending distally from the housing portion, the two body halves defining a longitudinal bore extending between proximal and distal ends thereof; and
an endoscope configured for insertion into the longitudinal bore of the obturator assembly, wherein at least a portion of the obturator assembly is transparent to permit visualization of tissue with the endoscope.

2. The visualization system according to claim 1, wherein the two body halves define a blunt end portion configured for separation of tissue planes and blunt dissection of cavity linings, and/or wherein the obturator assembly includes indicia to guide longitudinal alignment of the two body halves, and/or wherein the two body halves are substantially identical.

3. The visualization system according to claim 1 or claim 2, wherein the obturator assembly further includes a shrink wrap configured to secure the two body halves together as a single construct.

4. The visualization system according to claim 3, wherein the shrink wrap is wrapped around the elongate member portions of the two body halves.

5. The visualization system according to any preceding claim, wherein one of the two body halves includes a hook portion on a longitudinal edge thereof, and the other of the two body halves includes a slot portion configured to receive the hook portion therein to secure the two body halves together.

6. The visualization system according to any preceding claim, wherein the elongate member portion has a uniform thickness.

7. The visualization system according to any preceding claim, wherein the elongate member portions of the two body halves are transparent.

8. The visualization system according to any preceding claim, wherein the two body halves are entirely transparent.

9. The visualization system according to any preceding claim, further comprising a cannula assembly including a cannula housing and a cannula sleeve extending distally from the cannula housing, the cannula assembly defining a longitudinal channel extending between a proximal end of the cannula assembly to a distal end of the cannula assembly.

10. The visualization system according to claim 9, wherein the longitudinal channel is configured and dimensioned to receive at least a portion of the obturator assembly therethrough.

11. An obturator assembly comprising:
a first obturator body half having a first housing portion and a first elongate member portion extending distally from the housing portion;
a second obturator body half having a second housing portion and a second elongate member portion extending distally from the housing portion;
the first and second obturator body halves configured to be joined so as to form an obturator, wherein, when the first and second obturator body halves are joined, the first and second housing portions define a housing and the first and second elongate member portions define an elongate tubular member having a longitudinal bore extending between proximal and distal ends thereof, the housing and longitudinal bore configured to receive an endoscope.

12. The obturator assembly according to claim 11, wherein the elongate tubular member is at least partially transparent.

13. A method of manufacturing an obturator assembly comprising the steps of:
molding a first obturator body half having a first housing portion and a first elongate member portion extending distally from the housing portion;
molding a second obturator body half having a second housing portion and a second elongate member portion extending distally from the housing portion;
joining the first and second obturator body halves so as to form an obturator, wherein, when the first and second obturator body halves are joined, the first and second housing portions define a housing and the first and second elongate member portions define an elongate tubular member having a longitudinal bore extending between proximal and distal ends thereof.

14. The method according to claim 13, further comprising the steps of:
inserting an endoscope through the housing and the longitudinal bore.

15. The method according to claim 14, wherein the molding steps include molding the first and second elongate member portions from a material that is at least partially transparent.
